# EUROPÄISCHE PATENTANMELDUNG

(11) **EP 1 369 140 A2**
(43) Veröffentlichungstag der Anmeldung: **10.12.2003**
(21) Anmeldenummer: 03012338.4
(22) Anmeldetag: 30.05.2003
(51) Int. Cl.: A61M 15/00, B65D 83/16

(54) **Inhalationseinrichtung**

(30) Priorität: 06.06.2002 DE 10225059
(71) Anmelder: Gox GmbH, 89231 Neu-Ulm (DE)
(72) Erfinder: Berger, Oliver, 89231 Neu-Ulm (DE)
(74) Vertreter: Weber, Gerhard, Dipl.-Phys.

(57) **Zusammenfassung**

Inhalationseinrichtung mit einem Vorratsbehälter (VB) für ein Inhalationsgas und mit einem Inhalations-Aufsatz (AU), wobei der Vorratsbehälter (VB) an einer Entnahmeseite zur Entnahme von Inhalationsgas ein entgegen einer Schließkraft betätigbares Ventil (VE) aufweist, der Inhalations-Aufsatz (AU) eine einer Gesichtskontur angepasste Inhalationsmaske (IM) umfasst, handbetätigbare Bedienmittel (BH) zum Öffnen des Ventils (VE) entgegen der Schließkraft vorgesehen sind, der Aufsatz (AU) einen Grundkörper (HK) und relativ zu diesem bewegbare Bedienmittel (BH) enthält, der Grundkörper (HK) in der Gebrauchsposition auf dem Vorratsbehälter (VB) festlegbar ist, die Bedienmittel (BH) einen auserhalb vom Grundköper (HK) und Maske (IM) liegenden Griffabschnitt (GA) und einen bei Krafteinrichtung auf dem Griffabschnitt (GA) das Ventil (VE) öffnend betätigenden Druckabschnitt (DA) enthalten.

## Beschreibung

Die Erfindung betrifft eine Inhalationseinrichtung mit einem Vorratsbehälter für ein Inhalationsgas und mit einem Inhalations-Aufsatz.

Derartige Inhalationseinrichtungen sind insbesondere von Bedeutung für den mobilen Einsatz, beispielsweise bei überdurchschnittlicher körperlicher Beanspruchung, für Notfälle, in besonderer Umgebung usw.

Eine derartige Einrichtung ist beispielsweise aus der CH 685 678 A5 bekannt. Die bekannte Einrichtung umfasst einen dosenförmigen Vorratsbehälter, in welchem ein Inhalationsgas, beispielsweise Sauerstoff, in komprimierter Form gespeichert ist, und eine Inhaliermaske. Der Vorratsbehälter ist im wesentlichen zylindrisch und weist an einer Entnahmeseite in einem Dosendeckel integriert ein Entnahmeventil auf, welches entgegen einer rückstellenden Schließkraft durch Eindrücken in den Behälter geöffnet werden kann.

Die Inhaliermaske weist dem Vorratsbehälter abgewandt eine einer Gesichtskontur, insbesondere dem Mund-Nasen-Bereich eines Gesichts angepasste Form, insbesondere in Form einer mit ihrer konkaven Seite einen Inhalationsraum bildenden gewölbten Schale auf. Die Inhaliermaske kann in einer Gebrauchsposition auf das Ventil aufgesetzt werden. Durch Niederdrücken der Inhalationsmaske in Richtung des Vorratsbehälters entgegen der Schließkraft des Ventils wird das Ventil geöffnet und Inhalationsgas strömt aus dem Behälter in den Inhalationsraum der Inhaliermaske.

Der Gasstrom des Inhaliergases ist stark abhängig vom mit zunehmender Gasentnahme absinkenden Überdruck im Vorratsbehälter und von der Ventilstellung beim Eindrücken des Ventils. Um den Gasstrom unabhängig von diesen Parametern gleichmäßiger einzustellen ist bei der bekannten Anordnung eine Vorrichtung zum Ausgleichen des Gasstromes vorgesehen, welche eine Kammer mit einer frei beweglichen Kugel umfasst.

Der vorliegenden Erfindung liegt die Aufgabe zugrunde, Handhabung und/oder Einsatz einer derartigen handhaltbaren Inhalationseinrichtung zu verbessern.

Erfindungsgemäße Lösungen sind in den unabhängigen Ansprüchen beschrieben. Die abhängigen Ansprüche enthalten vorteilhafte Ausgestaltungen und Weiterbildungen der Erfindung.

Die relativ zu dem Grundkörper des Aufsatzes beweglichen Bedienmittel ermöglichen die Festlegung des Grundkörpers auf dem Vorratsbehälter in einer definierten, für die Entnahme von Gas nicht mehr zu verändernden Gebrauchsposition. Die Bedienmittel weisen einen leicht erreichbaren Griffabschnitt außerhalb von Grundkörper und Maske auf, welcher vorzugsweise in der Gebrauchsposition so angeordnet ist, dass er mit einem Finger einer den Vorratsbehälter bei der Entnahmeseite umgreifenden Hand erreichbar ist. Vorzugsweise liegt der Griffabschnitt auf der dem Benutzer zugewandten Seite der Inhalationseinrichtung. Die Inhalationseinrichtung ist dabei vorteilhafterweise mit nur einer Hand benutzbar.

Die Bedienmittel enthalten vorteilhafterweise einen um ein Hebelgelenk am Grundkörper schwenkbaren Hebel, bei welchem der auf das Ventil öffnend einwirkende Druckabschnitt vorzugsweise zwischen dem Hebelgelenk und dem Griffabschnitt liegt. Die Schwenkachse des Hebels verläuft vorteilhafterweise in einer zur Bewegungsrichtung des Ventils senkrechten Richtung. Die Bewegungsrichtung des Ventils beim öffnen liegt dann vorzugsweise tangential zur Schwenkbewegung des Druckabschnitts des Hebels.

Durch einen größeren Abstand des Griffabschnitts vom Hebelgelenk als der Druckabschnitt ergibt sich vorteilhafterweise durch die reduzierte Bedienkraft auf dem Griffabschnitt eine erleichterte Handhabung und durch den gegenüber dem kurzen Ventilweg längeren Bedienung des Griffabschnitts zusätzlich die Möglichkeit, den Gasstrom variabel zu dosieren und insbesondere auch für die Ausatmungsphase zu unterbrechen. Dies führt zu einem effektiven Einsatz des Inhalationsgases und damit zu einer größeren Nutzungsdauer eines Vorratsbehälters mit gegebenem Volumen und Anfangsdruck. Die geringere Bedienkraft und der längere Bedienweg wirken bei der Dosierbarkeit des Gasstroms besonders vorteilhaft zusammen und erübrigen dadurch vorteilhafterweise auch eine Ausgleichsvorrichtung wie beim Stand der Technik. Das Hebelübersetzungsverhältnis ist vorzugsweise größer als zwei.

Der Einsatz federbelasteter Bedienhebel zur Entnahme von Medien aus Vorratsbehältern ist an sich bekannt, beispielsweise bei Pumpzerstäubern oder Ölspendern. Eine Anregung zum Einsatz eines Hebels als Bedienmittel in einer Inhalationseinrichtung der beschriebenen Art ist dadurch aber nicht angeregt.

Der Vorratsbehälter ist vorzugsweise in der an sich bekannten und auch beim Stand der Technik eingesetzten Form einer überwiegend kreiszylindrischen Dose ausgeführt, welche an der Entnahmeseite mit einem Deckel verschlossen ist, in welchem das Ventil integriert ist. Die Gasauslassöffnung ist typischerweise durch ein auf der Mittelachse der kreiszylindrischen Dose liegendes Rohrstück gegeben, welches zum Öffnen entgegen der Schließkraft entlang der Mittelachse in Richtung des Behälters gedrückt wird.

Gemäß einer vorteilhaften Ausführung wird das bei Öffnen des Ventils ausströmende Gas aus der typischerweise mit der Betätigungsrichtung des Ventils zusammenfallenden Haupt-Ausströmrichtung umgelenkt, wozu vorteilhafterweise in den Bedienmitteln bei dem Druckabschnitt Leitstrukturen zur Umlenkung quer zu dieser Haupt-Ausströmrichtung vorgesehen sind. Diese können insbesondere eine in Verlängerung der Haupt-Ausströmrichtung im wesentlichen senkrecht zu dieser ausgerichtete Prallfläche umfassen. Vorzugsweise wird das so umgelenkte Gas durch von dem Ventil quer zur Haupt-Ausströmrichtung beabstandete Austrittsöffnungen in den Inhalationsraum geleitet. Diese sind vorteilhafterweise flächig verteilt. Durch einen über alle Austrittsöffnungen summierten Gesamtöffnungsquerschnitt, der wesentlich größer ist als der Ausströmquerschnitt des Ventils, kann vorteilhafterweise die Ausströmgeschwindigkeit des Gases in den Inhalationsraum reduziert werden.

Die rückstellende Schließkraft des Ventils wirkt vorteilhafterweise auch rückstellend auf die Bedienmittel, welche dadurch ohne eigene Rückstellkraft nicht selbsthaltend sind, so dass bei Wegnahme der Betätigungskraft das Ventil im wesentlichen unverzögert schließt.

Der Grundkörper des Aufsatzes ist in der Gebrauchsposition vorteilhafterweise lösbar auf dem Vorratsbehälter in definierter Position festgelegt. Günstigerweise weist der Grundkörper hierfür Gegenstrukturen zu einer Reliefstruktur des Vorratsbehälters um das Ventil auf. Solche Reliefstrukturen sind an sich bei üblichen Ventildeckeln im Regelfall bereits vorhanden und können auch einen Bördelrand, an welchem der Deckel mit dem Behälter gasdicht verbunden ist, umfassen.

Der Grundkörper wird vorzugsweise in einer mit der Betätigungsrichtung des Ventils zusammenfallenden Steckrichtung auf dem Vorratsbehälter aufgesetzt, wobei zum einen die Gegenstrukturen des Grundkörpers in die Reliefstruktur des Vorratsbehälters eingreifen und gegen seitliche Verschiebung zentriert festlegen. Zusätzlich zu den Gegenstrukturen können Abstützstrukturen in größerem Radius vorgesehen sein, welche den Aufsatz weiter gegen Verkippen stabilisieren. Das Aufsetzen ist vorteilhafterweise durch Anschlag des Grundkörpers bzw. der an diesem ausgebildeten Strukturen am Vorratsbehälter begrenzt. Der Aufsatz befindet sich danach in einer von der Ventilstellung unabhängigen stabilen Gebrauchsposition, welche er auch beim Öffnen und Schließen des Ventils im wesentlichen beibehält. Ein Festhalten gegen Abziehen des Aufsatzes vom Vorratsbehälter kann beispielsweise durch Klebemittel oder durch Formschluss zwischen behälterseitigen und aufsatzseitigen Verriegelungselementen einschließlich Verschnappungen erfolgen. Bevorzugt ist jedoch eine Ausführung, bei welcher die Gegenstrukturen des Grundkörpers klemmend in die Reliefstruktur des Vorratsbehälters eingreifen und kraftschlüssig auf diesen gehalten sind. Durch Überwinden der Haftreibungskraft der Klemmung in abziehender Richtung kann der Aufsatz wieder zerstörungsfrei vom Vorratsbehälter abgenommen werden. Da das Aufsetzen des Aufsatzes bis zum Anschlag auf dem Vorratsbehälter ohne Betätigung des Ventils erfolgt, tritt die Ventilschließkraft nicht als abziehende Kraft oder als Gegenkraft beim Aufsetzen in Erscheinung.

Die beim Betätigen des Ventils mittels der Bedienmittel u. U. als abziehend gerichtete Komponente auftretende Schließkraft des Ventils wird vorteilhafterweise teilweise kompensiert durch eine Ausrichtung des Griffabschnitts in der Weise, dass die Kraft auf dem Griffabschnitt in einer zur Richtung der Ventilschließkraft annähernd entgegengesetzten Richtung ausgeübt wird.

Der Aufsatz kann vorteilhafterweise zusätzlich zu der Gebrauchsposition mit auf das Ventil einwirkenden Bedienmitteln und mit dem Behälter abgewandtem Inhalationsraum der Maske in zum Stand der Technik ähnlicher Weise auch in einer Nichtgebrauchsposition auf die Entnahmeseite des Vorratsbehälters aufsetzbar sein und in dieser Position den Entnahmebereich des Behälters mit der Maske umgeben. Hierfür sind vorzugsweise im Innern der Maske an dem Grundkörper zweite Gegenstrukturen ausgebildet, welche, z. B. klemmend, auf die Reliefstruktur des Vorratsbehälters aufgesetzt werden können. Die Bedienmittel sind für die Nichtgebrauchsposition vorteilhafterweise unter Beibehaltung der Gelenkverbindung am Grundkörper in eine andere Lage, insbesondere nahe der Innenwand der Inhalationsmaske verschwenkbar.

Eine andere vorteilhafte Maßnahme für eine Inhalationseinrichtung mit einem Vorratsbehälter für ein Inhalationsgas und mit einer Inhalationsmaske besteht darin, im Strömungsweg des Inhalationsgases zwischen dem Ausgang des Behälterventils und dem Inhalationsraum eine Aufnahme für eine Anreicherungssubstanz vorzusehen. Eine solche Aufnahme ist vorzugsweise als kammerartige Aufweitung des Strömungswegs ausgeführt, in welche auswechselbar ein Trägerkörper einsetzbar ist, welcher die Anreicherungssubstanz enthält und beim Durchströmen von Inhalationsgas kleine Mengen an dem Gasstrom abgibt.

Der Trägerkörper kann beispielsweise ein offenporiger Festkörper sein, welcher mit der Anreicherungssubstanz getränkt oder beschichtet ist Bevorzugt als Trägerkörper ist ein Faserkörper, welcher vom Inhalationsgas durchströmt werden kann und an oder in dessen Fasern Anreicherungssubstanz eingelagert ist. Die Aufnahme kann auch lediglich als Bypass von einem Teil des gesamten Gasstroms durchströmt sein.

Die Aufnahme kann besonders vorteilhaft in den Bedienmitteln, insbesondere im Bereich des Druckabschnitts um das Ventil ausgebildet sein. Die Bedienmittel können hierfür vorteilhafterweise eine Oberschale und eine Unterschale enthalten, welche zusammengesetzt die Aufnahme bilden und zerstörungsfrei zum Austausch des Trägerkörpers geöffnet werden können. Oberschale und Unterschale sind vorzugsweise in einem einstückigen Kunststoffkörper über ein Filmscharnier verbunden.

Die Anreicherungssubstanz kann insbesondere ein Duftstoff und/oder eine anregende Substanz und/oder eine medizinisch wirksame Substanz sein. Die Anreicherungssubstanz kann in der Aufnahme auch in einer chemischen Vorform vorliegen und mit dem Inhalationsgas zur Wirksubstanz reagieren. Die Anreicherungssubstanz kann auch verschiedene Stoffe enthalten, beispielsweise verschiedene sich ergänzende Wirkstoffe oder einen Duftstoff zu einem medizinisch wirksamen Stoff mit unangenehmem Eigengeruch.

Die Anreicherungssubstanz in der Aufnahme kann beispielsweise durch individuell wählbare Duftnoten die Akzeptanz einer verordneten Inhalation verbessern, insbesondere bei einem Inhalationsgas mit unangenehmem Eigengeruch. Insbesondere kann auch durch die Aufnahme dem Inhalationsgas, insbesondere medizinisch wirksamem Sauerstoff, ein medizinisch wirksamer Stoff etc. in genau dosierbarer Menge und zur schnelleren Resorption mit effizientem Einsatz des Stoffes zugefügt werden. Dabei kann der Aufbau der Inhalationseinrichtung einfach gehalten werden.

Die Erfindung ist nachfolgend anhand bevorzugter Ausführungsbeispiele unter Bezugnahme auf die Abbildungen noch eingehend veranschaulicht. Dabei zeigt
Fig. 1 ein Schnittbild durch eine Inhalationseinrichtung,
Fig. 2 einen Kunststoffkörper für Bedienmittel in offenem Zustand,
Fig. 3 die Inhalationseinrichtung nach Fig. 1 in einer Nichtgebrauchsstellung.

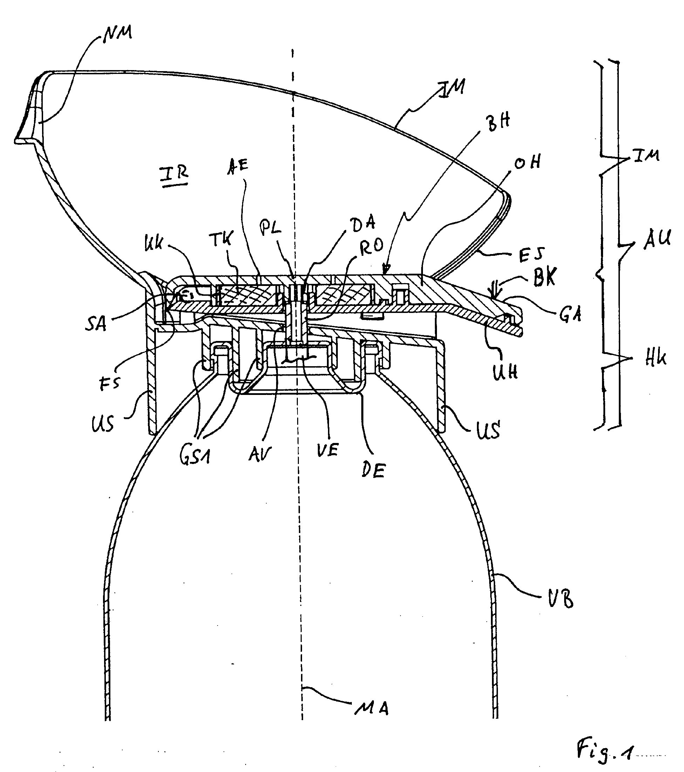

Die in Fig. 1 skizzierte Inhaliereinrichtung legt, ohne Einschränkung hierauf, einen im wesentlichen um eine Mittelachse MA kreiszylindrischen Dosenkörper als Vorratsbehälter VB zugrunde. Der Vorratsbehälter ist an einer Stirnseite durch einen Deckel DE mit einem Ventil VE abgeschlossen. Aufbauformen eines solchen Ventildeckels sind allgemein bekannt und das Ventil VE ist daher der Übersichtlichkeit halber nur durch das den Ventilausgang bildende Rohrstück RO angedeutet. Zur Entnahme von mit Überdruck im Vorratsbehälter gespeichertem Inhalationsgas ist das Rohrstück entgegen einer Schließkraft in Richtung der Mittelachse zum Behälter hin zu drücken. Ab einer durch die Konstruktion vorgegebenen Eindrucktiefe strömt Gas aus dem Behälterinneren durch das Rohrstück nach außen, wobei die Haupt-Ausströmrichtung in Richtung der Mittelachse MA verläuft und durch den geringen Querschnitt des Rohrstücks ein für Inhalationszwecke häufig als unangenehm empfundener Gasstrahl entstehen kann.

Der Deckel weist in an sich üblicher Weise eine kreissymmetrische Reliefstruktur mit Erhebungen, Vertiefungen, Flanken, Bördelrand etc. auf. Ein Aufsatz AU mit einem Grundkörper HK und einer Inhalationsmaske IM weist an einer der Inhalationsmaske abgewandten Seite erste Gegenstrukturen GS1, z. B. in Form von Ringstegen und Tiefenanschlägen auf, mittels welcher der Aufsatz mit vom Vorratsbehälter weg weisender Inhalationsmaske klemmend auf dem Vorratsbehälter aufgesetzt und dort kraftschlüssig gehalten werden kann. Beim Aufstecken des Aufsatzes auf den Vorratsbehälter wird das Rohrstück durch eine Aussparung AV in einer Fläche des Grundkörpers ohne Betätigen des Ventils hindurchgeführt und ragt über diese Fläche hinaus. Die Aussparung AV ist so weit gegenüber dem Durchmesser des Rohrstücks vergrößert, dass eine achsiale Verschiebung des Rohrstücks zur Betätigung des Ventils nicht behindert ist.

Der Aufsatz AU nimmt nach dem Aufstecken auf dem Vorratsbehälter bis zum Anschlag eine Gebrauchsstellung ein, welche für geöffnetes und geschlossenes Ventil im wesentlichen erhalten bleibt. Zum Kompensieren von beim Betätigen des Ventils über nachfolgend noch näher erläuterte Bedienmittel BH eventuell auftretende Kippmomente können, insbesondere auf der Seite des Kraftangriffs BK auf einen Griffabschnitt GA der Bedienmittel, im skizzierten Beispiel rechts und/oder auf der entgegen gesetzten Seite zusätzliche Abstützelemente vorgesehen sein. Solche Abstützelemente sind im Beispielsfall als ein gegenüber den Gegenstrukturen GS in größerem Abstand von der Mittelachse MA verlaufender Ringsteg US ausgebildet, welcher auf der Seite des Griffabschnitts GA durch Abstützen auf dem Vorratsbehälter und/oder auf der entgegen gesetzten Seite durch Greifen mit einem Finger einer den Vorratsbehälter in dem skizzierten Bereich bei der Entnahmeseite umgreifenden Hand Kippmomenten entgegen wirken kann. Im Beispielsfall ist der Griffabschnitt GA durch den Daumen einer Benutzerhand bedienbar.

Als Bedienmittel ist im skizzierten bevorzugten Beispielsfall ein um eine Schwenkachse SA schwenkbarer Bedienhebel BH in einem Schwenkgelenk im Hauptkörper HK gelagert. Ein Griffabschnitt GA des Bedienhebels BH ragt über Grundkörper und Inhalationsmaske seitlich hinaus. Die Schwenkachse SA ist senkrecht zur Zwischenebene der Fig. 1 und damit auch senkrecht zur in Richtung der in der Zeichenebene liegenden Mittelachse MA verlaufenden Verschiebbarkeit des Rohrstücks RO. Ein Druckabschnitt DA, mit welchem der Hebel BH auf dem Ausgang des Rohrstücks aufliegt, liegt zwischen dem Griffabschnitt und dem Schwenkgelenk. Das Schwenkgelenk liegt bezüglich der Richtung der Mittelachse MA ungefähr auf der Höhe des Ausgangs des Rohrstücks, so dass die Bewegungsrichtung des Ventils annähernd tangential zu der Schwenkbewegung des Druckabschnitts verläuft. Der Griffabschnitt liegt vorzugsweise gleichfalls annähernd auf Höhe des Rohrstücks RO.

Die Inhalationsmaske IM weist in an sich gebräuchlicher Weise die Form einer offenen gewölbten Schale auf, die einen Inhalationsraum IR umgibt, und ist der Kontur eines Gesichtes angepasst. Die Inhalationsmaske ist nicht drehsymmetrisch und weist an einer Seite eine Mulde NM auf, in welcher beim Inhalationsvorgang der Nasenrücken des Benutzers liegt. Die Unterteilung des Aufsatzes in einen Grundkörper und eine Inhalationsmaske nach Fig. 1 ist funktionell und nicht als strenge geometrische Trennlinie zu verstehen. Inhalationsmaske und Grundkörper sind vorzugsweise einstückig als Kunststoff-Formteil ausgeführt.

Der Bedienhebel BH weist vorteilhafterweise bei dem Druckabschnitt DA in Verlängerung des Rohrstücks RO eine den direkten Gasausstrom in Richtung der Mittelachse behindernden Prallplattenabschnitt PL auf, durch welchen ausströmendes Gas quer zur Haupt-Abströmrichtung umgeleitet wird.

Der Bedienhebel BH besteht vorteilhafterweise aus einer Oberschale OH und einer Unterschale UH, welche in Gebrauchsposition aneinanderliegen und einen Strömungsabschnitt für das ausströmende Inhalationsgas bilden. Insbesondere kann zwischen Oberschale und Unterschale das Rohrstück RO und den auf dessen Rand abgestützten Druckabschnitt umgebend eine kammerartige Aufnahme KK gebildet sein, welche mit einer Mehrzahl flächig verteilter Austrittsöffnungen AE und/oder einer gegenüber dem Querschnitt des Rohrstücks RO wesentlich größeren Austrittsöffnung in den Inhalationsraum mündet. Neben einer Verteilung des Gasstroms kann die Aufnahme insbesondere zum Einsetzen eines Trägerkörpers TK dienen, welcher eine Anreicherungssubstanz an das durchströmende Inhalationsgas abgibt. Zum Auswechseln des Trägerkörpers sind die Oberschale und Unterschale vorteilhafterweise zerstörungsfrei voneinander abhebbar, insbesondere durch relatives Verschwenken um ein verbindendes Filmscharnier. Vorzugsweise kann der Bedienhebel dabei mit dem Grundkörper verbunden bleiben.

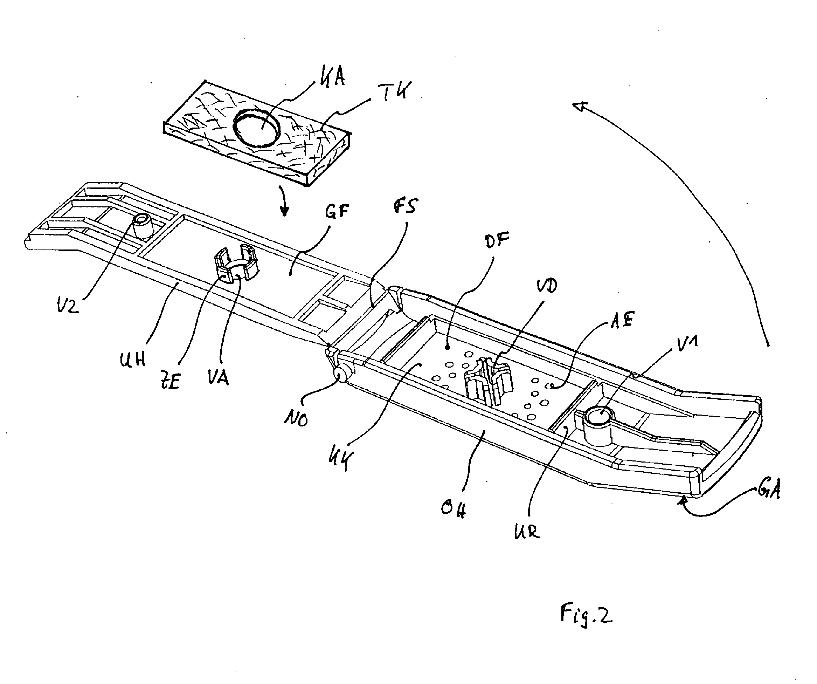

In Fig. 2 ist eine besonders vorteilhafte Ausführung eines als einstückiges Kunststoffteil in Spritzgußtechnik herstellbaren Bedienhebels mit in aufgeklappter Position über ein Filmscharnier FS verbundener Oberschale OH und Unterschale UH skizziert.

In der Oberschale OH sind in einer Deckfläche DF, welche im Gebrauchszustand dem Inhalationsraum zugewandt ist eine Mehrzahl von Austrittsöffnungen AE flächig verteilt angeordnet. Ein aus mehreren Stegen mit Strömungslücken bestehendes Druckstück VD ist am Druckabschnitt des Hebels an der Deckfläche angeformt. Die Stege liegen in der Gebrauchsstellung an dem Rohrrand des Rohrstücks RO an. Der Raum um das Druckstück ist in Form einer Aufnahmekammer seitlich begrenzt durch die Seitenwand der Oberschale OH und Kammerwände KR.

An der Oberschale sind seitlich abstehende Nocken NO angeformt, welche zur gelenkigen Lagerung des Bedienhebels in Aussparungen des Grundkörpers eingreifen. An dem den Nocken abgewandten Ende der Oberschale ist der Griffabschnitt GA ausgebildet.

Die Unterschale UH weist eine Grundfläche GF auf, welche im zusammengeklappten Zustand die Aufnahmekammer nach unten abschließt. Teile der seitlichen Begrenzung der Kammer können als Aussteifungsstege auch an der Unterschale ausgebildet sein. In der Grundfläche GF der Unterschale befindet sich eine Aussparung, welche in der Gebrauchsstellung das Rohrstück RO mit geringem Abstand umschließt. An der Grundfläche der Unterschale können zusätzliche Zentrierelemente ZE vorgesehen sein.

Ein Trägerkörper TK für eine Anreicherungssubstanz kann in die Aufnahmekammer eingesetzt werden und weist hierfür eine zentrale Aussparung KA, durch welche die Zentrierelemente ZE und/oder das Druckstück VD hindurchragen, auf.

Oberschale und Unterschale werden zur Bildung des Bedienhebels relativ zueinander um das Filmscharnier verschwenkt und über korrespondierende Verschlusselemente V1, V2 miteinander verbunden. Die Verbindung über die Verschlusselemente V1, V2 ist zerstörungsfrei lösbar um den Trägerkörper TK austauschen zu können. Der Trägerkörper ist vorzugsweise gasdurchlässig und besteht vorzugsweise aus Fasermaterial mit eingelagerter Anreicherungssubstanz.

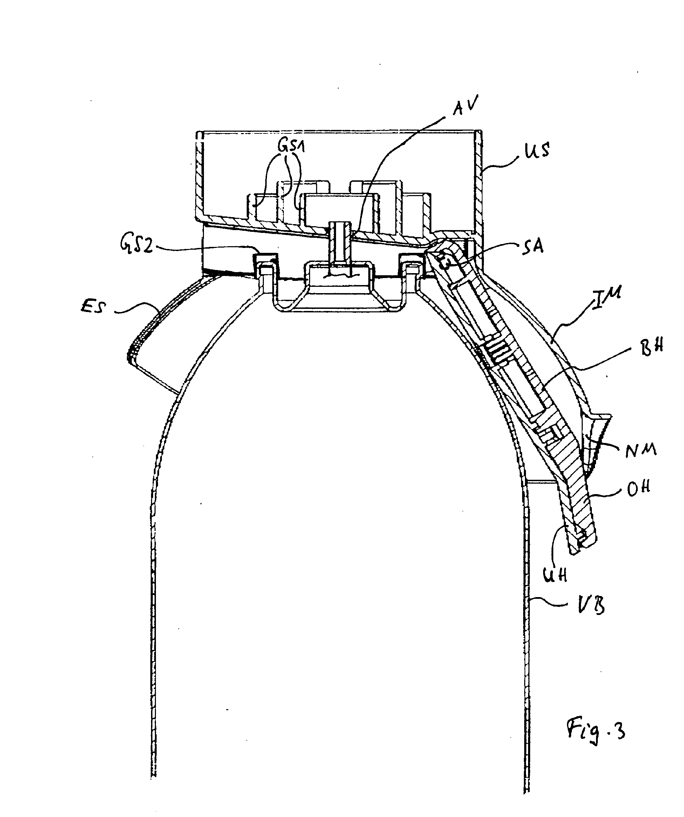

Fig. 3 zeigt eine Nichtgebrauchsstellung der in Fig. 1 skizzierten Inhalationseinrichtung, bei welcher der Aufsatz über zweite Gegenstrukturen GS2 auf der Reliefstruktur des Vorratsbehälters gehalten ist und die Inhalationsmaske die Entnahmeseite des Vorratsbehälters umgibt. Der Bedienhebel BH ist hierfür ohne Lösen der Gelenkverbindung mit dem Grundkörper um die Schwenkachse in eine in Fig. 3 skizzierte Lage innerhalb des Aufsatzes AU verschwenkbar und ist in dieser Lage im wesentlichen spielfrei gehalten. Zur Verschwenkung des Hebels BH zwischen den Lagen nach Fig. 1 und Fig. 3 kann die Inhalationsmaske einen Einschnitt ES aufweisen.

Die vorstehend und die in den Ansprüchen angegebenen sowie die den Abbildungen entnehmbaren Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar. Insbesondere kann der Bedienhebel auch auf der von dem Benutzer wegweisenden Seite über den Grundkörper hinausragen und mit dem Zeigefinger einer den Vorratsbehälter umgreifenden Hand bedienbar sein.

## Patentansprüche

1. Inhalationseinrichtung mit einem Vorratsbehälter für ein Inhalationsgas und mit einem Inhalations-Aufsatz, wobei
a) der Vorratsbehälter an einer Entnahmeseite zur Entnahme von Inhalationsgas ein entgegen einer Schließkaft betätigbares Ventil aufweist,
b) der Inhalations-Aufsatz eine einer Gesichtskontur angepasste Inhalations-Maske umfasst, welche einen Inhalationsraum bildet, welcher in einer Inhalationsposition des Aufsatzes von dem Vorratsbehälter weg gerichtet ist.
c) handbetätigbare Bedienmittel zum Öffnen des Ventils entgegen der Schließkraft vorgesehen sind,
**dadurch gekennzeichnet, dass** der Aufsatz einen Grundkörper und relativ zu diesem bewegbare Bedienmittel enthält, dass der Grundkörper in der Gebrauchsposition auf dem Vorratsbehälter festlegbar ist, dass die Bedienmittel einen außerhalb vom Grundkörper und Maske liegenden Griffabschnitt und einen bei Krafteinwirkung auf den Griffabschnitt das Ventil öffnend betätigenden Druckabschnitt enthalten.

2. Inhalationseinrichtung nach Anspruch 1, **dadurch gekennzeichnet, dass** der Griffabschnitt im Bewegungsbereich eines Fingers einer den Vorratsbehälter umgreifenden Hand liegt.

3. Inhalationseinrichtung nach Anspruch 2, **dadurch gekennzeichnet, dass** der Griffabschnitt auf den Benutzer zuweist und im Bewegungsbereich des Daumens einer den Vorratsbehälter umgreifenden Hand liegt.

4. Inhalationseinrichtung nach einem der Ansprüche 1 bis 3, **dadurch gekennzeichnet, dass** die Bedienmittel einen in einem Hebelgelenk gelagerten Hebel enthalten.

5. Inhalationseinrichtung nach Anspruch 4, **dadurch gekennzeichnet, dass** der Druckabschnitt zwischen Griffabschnitt und Hebelgelenk liegt.

6. Inhalationseinrichtung nach einem der Ansprüche 1 bis 5, **dadurch gekennzeichnet, dass** bei dem Druckabschnitt der Bedienmittel Leitstrukturen zur Umlenkung des aus dem Ventil ausströmenden Gases quer zur Haupt-Ausströmrichtung aus dem Ventil aufweisen.

7. Inhalationseinrichtung nach Anspruch 6, **dadurch gekennzeichnet, dass** die-Leitstrukturen in eine Kammer münden, welche Austrittsöffnungen zum Inhalationsraum der Maske aufweist.

8. Inhalationseinrichtung nach Anspruch 7, **dadurch gekennzeichnet, dass** die Austrittsöffnungen gegen den Ventilausgang seitlich versetzt sind.

9. Inhalationseinrichtung nach einem der Ansprüche 1 bis 7, **dadurch gekennzeichnet, dass** die Rückstellkraft des Ventils auch rückstellend auf die Bedienmittel wirkt.

10. Inhalationseinrichtung nach einem der Ansprüche 1 bis 9, **dadurch gekennzeichnet, dass** in der Gebrauchsposition der Aufsatz lösbar auf dem Vorratsbehälter festlegbar ist.

11. Inhalationseinrichtung nach Anspruch 10, **dadurch gekennzeichnet, dass** der Vorratsbehälter an der Entnahmeseite um das Ventil eine Reliefstruktur und der Grundkörper des Aufsatzes dazu Gegenstrukturen aufweisen.

12. Inhalationseinrichtung nach Anspruch 11, **dadurch gekennzeichnet, dass** der Grundkörper mit den in einer Befestigungsrichtung in die Reliefstruktur des Vorratsbehälters eingreifenden Gegenstrukturen auf dem Vorratsbehälter lösbar festlegbar ist.

13. Inhalationseinrichtung nach einem der Ansprüche 1 bis 12, **dadurch gekennzeichnet, dass** die Krafteinwirkung auf den Griffabschnitt der Bedienmittel zur Ventilbetätigung im wesentlichen in Befestigungsrichtung erfolgt.

14. Inhalationseinrichtung nach einem der Ansprüche 1 bis 13, **dadurch gekennzeichnet, dass** der Aufsatz neben der Gebrauchsposition zusätzlich in einer Nichtgebrauchsposition auf dem Vorratsbehälter festlegbar ist.

15. Inhalationseinrichtung nach Anspruch 14, **dadurch gekennzeichnet, dass** die Bedienmittel für die Festlegung des Aufsatzes in der Nichtgebrauchsposition in entsprechender Weise innerhalb des Aufsatzes in eine Nichtgebrauchslage verschwenkbar sind.

16. Handhaltbare Inhalationseinrichtung mit einem Vorratsbehälter für ein Inhalationsgas und mit einer Inhalationsmaske, wobei
a) der Vorratsbehälter an einer Entnahmeseite ein entgegen einer Schließkraft zur Entnahme von Inhalationsgas betätigbare Ventil aufweist,
b) die Inhalationsmaske in einer Inhalationsposition an der Entnahme-Seite mit einer Halbschale, die einen einer Gesichtskontur angepassten Inhalationsraum bildet, vom Vorratsbehälter wegweist,
insbesondere nach einem der weitergehenden Ansprüche, **dadurch gekennzeichnet, dass** im Strömungsweg zwischen dem Ausgang des Ventils und dem Inhalationsraum eine Aufnahme für eine Anreicherungssubstanz vorgesehen ist.

17. Inhalationseinrichtung nach Anspruch 16, **dadurch gekennzeichnet, dass** ein Trägerkörper für die Anreicherungssubstanz in die Aufnahme einlegbar ist und dort die Anreicherungssubstanz an durchströmendes Inhalationsgas abgibt.

18. Inhalationseinrichtung nach Anspruch 17, **dadurch gekennzeichnet, dass** der Trägerkörper als offenporiger mit Anreicherungssubstanz getränkter Formkörper ausgeführt ist.

19. Inhalationseinrichtung nach Anspruch 17, **dadurch gekennzeichnet, dass** der Trägerkörper als Faserkörper, insbesondere als Vlies, Filz oder Gewebe ausgeführt ist.

20. Inhalationseinrichtung nach einem der Ansprüche 16 bis 19, **gekennzeichnet durch** einen Duftstoff und/oder einen medizinisch wirksamen Stoff als Anreicherungssubstanz.

21. Inhalationseinrichtung nach einem der Ansprüche 1 bis 20, **dadurch gekennzeichnet, dass** die Aufnahme für die Anreicherungssubstanz in den Bedienmitteln ausgebildet ist.

22. Inhalationseinrichtung nach einem der Ansprüche 1 bis 21, **dadurch gekennzeichnet, dass** die Bedienmittel aus einer Oberschale und einer Unterschale aufgebaut sind.

23. Inhalationseinrichtung nach Anspruch 22, **dadurch gekennzeichnet, dass** Oberschale und Unterschale in einem einstückigen Kunststoffkörper über ein Filmscharnier verbunden sind.
